# EUROPÄISCHE PATENTSCHRIFT

(11) **EP 1 222 162 B1**
(45) Veröffentlichungstag und Bekanntmachung des Hinweises auf die Patenterteilung: **06.05.2004**
(21) Anmeldenummer: 00971328.0
(22) Anmeldetag: 06.10.2000
(51) Int. Cl.: C07C 257/18, A61K 31/155, A61P 11/06

(54) **NEUER LTB4-ANTAGONIST, VERFAHREN ZU DESSEN HERSTELLUNG UND DESSEN VERWENDUNG ALS ARZNEIMITTEL**
NOVEL LTB4-ANTAGONIST, METHOD FOR THE PRODUCTION THEREOF AND ITS USE AS A MEDICAMENT
NOUVEL ANTAGONISTE DE LTB4, PROCEDE PERMETTANT DE LE PREPARER ET SON UTILISATION COMME MEDICAMENT

(30) Priorität: 07.10.1999 DE 19948428
(43) Veröffentlichungstag der Anmeldung: 17.07.2002
(73) Patentinhaber: Boehringer Ingelheim Pharma GmbH & Co.KG, 55218 Ingelheim am Rhein (DE)
(72) Erfinder: ANDERSKEWITZ, Ralf, 88471 Lauphelm (DE)
(86) Internationale Anmeldenummer: PCT/EP2000/009793
(87) Internationale Veröffentlichungsnummer: WO 2001/025186

(56) Entgegenhaltungen:
- WO-A-93/16036
- WO-A-98/11062

## Beschreibung

Die vorliegende Erfindung betrifft einen neuen LTB₄-Antagonist, Verfahren zu dessen Herstellung sowie dessen Verwendung als Arzneimittel.

LTB₄-Antagonisten sind aus dem Stand der Technik bekannt. So werden durch die Internationale Patentanmeldung WO 98/11062 Benzamidinderivate mit vorstehend genannter pharmakologischer Aktivität offenbart.

Überraschenderweise wurde gefunden, daß der erfindungsgemäße, neue LTB₄-Antagonist gegenüber aus dem Stand der Technik bekannten Verbindungen überlegene Eigenschaften besitzt. In diesem Zusammenhang seien die außergewöhnlich hohe in vitro- und in vivo-Aktivität als auch die überraschend hohe metabolische Stabilität des erfindungsgemäßen, neuen LTB₄-Antagonisten genannt.

Bei dem erfindungsgemäßen LTB₄-Antagonist handelt es sich um die Verbindung der Formel (I) 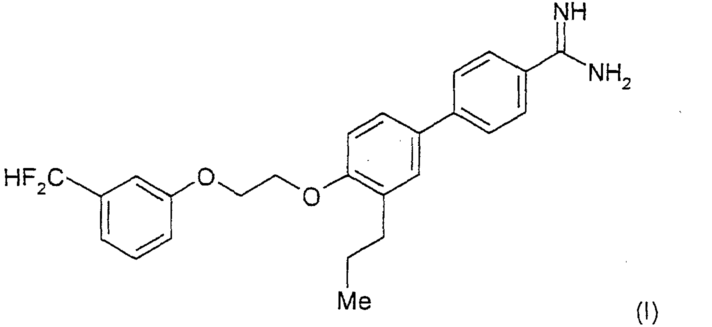 gegebenenfalls in Form ihrer pharmakologisch unbedenklichen Säureadditionssalze. Wie vorstehend genannt, kann die Verbindung der Formel (I) in ihre Salze, insbesondere für die pharmazeutische Anwendung, in ihre physiologisch und pharmakologisch verträglichen Salze mit einer anorganischen oder organischen Säure, überführt werden. Als Säuren kommen hierfür beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden.

Wie gefunden wurde, zeichnet sich die Verbindung der Formel I durch vielfältige Anwendungsmöglichkeiten auf therapeutischem Gebiet aus. Hervorzuheben sind solche Anwendungsmöglichkeiten, für welche die LTB₄-rezeptorantagonistischen Eigenschaften eine Rolle spielen.

Hier sind insbesondere zu nennen: Arthritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, cystische Fibrose, Alzheimer-Krankheit, Schock, Reperfusionsschäden/Ischämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB₄ oder eines anderen Moleküls (beispielsweise 12-HETE) mit dem LTB₄-Rezeptor einen Einfluß auf die Zell-Proliferation hat (etwa chronische myelozytische Leukämie).

Die neue Verbindung kann auch in Kombination mit anderen Wirkstoffen angewendet werden, etwa solchen, die für dieselben Indikationen Verwendung finden, oder z.B. mit Antiallergika, Sekretolytika, β₂-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Zur pharmakolgischen und biochemischen Untersuchung der Wirkungsverhältnisse eignen sich Tests, wie sie beispielsweise in der WO 93/16036, S 15 bis 17 - auf die hier inhaltliche Bezug genommen wird - offenbart sind.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen:

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

Die neue Verbindung kann in Analogie zu aus dem Stand der Technik bekannten Syntheseverfahren zur Herstellung strukturell vergleichbarer Benzamidinderivate erhalten werden. An dieser Stelle wird besonders hingewiesen auf die durch die Internationale Patentanmeldung WO 98/11062 offenbarten Herstellungsverfahren, welche u.a. den synthetischen Zugang zu Benzamidin-Derivaten durch Reduktion der entsprechenden Amidoxime, durch Amminolyse der entsprechenden Iminoester sowie durch Umsetzung geeignet substituierter Phenole mit durch eine nukleofuge Abgangsgruppe substituierten Aryloxyalkylen im Sinne einer Williamson-Ethersynthese beschreiben.

Alternativ dazu ist die erfindungsgemäße Verbindung der Formel (I) beispielsweise zugänglich durch Umsetzung des Nitrils der Formel (II) 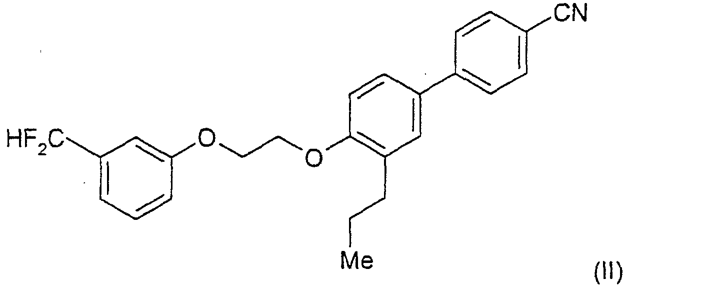 mit Li-Hexamethyldisilazan. Für die Reaktion eignen sich unpolare und polare aprotische Lösungsmittel wie Toluol, Ether, Tetrahydrofuran bei Temperaturen von -80°C bis 120°C. Zur Abspaltung der Silylgruppen werden anorganische und organische Säuren verwendet, wie HCl, HBr, H₂SO₄, Sulfonsäuren wie p-Toluolsulfonsäure Benzolsulfonsäure oder Methansulfonsäure sowie Carbonsäuren wie Ameisensäure, Essigsäure oder Trifluoressigsäure bei Temperaturen von 0°C bis 100°C.

Die erfindungsgemäße Verbindung ist ausgehend von aus dem Stand der Technik bekannten Verbindungen u.a. nach dem im folgenden Syxnthesebeispiel beschriebenen Verfahren herstellbar. Verschiedenartige, andere Ausgestaltungen des Verfahren werden für den Fachmann aus der vorliegenden Beschreibung sowie aus der vorstehend genannten internationalen Patentanmeldung WO 98/11062, auf die diesbezüglich inhaltlich in vollem Umfang Bezug genommen wird, ersichtlich. Es wird ausdrücklich darauf hingewiesen, daß das nachfolgende Synthesebeispiel lediglich zum Zweck der Erläuterung vorgesehen und nicht als Einschränkung der Erfindung anzusehen ist.

### Synthesebeispiel:

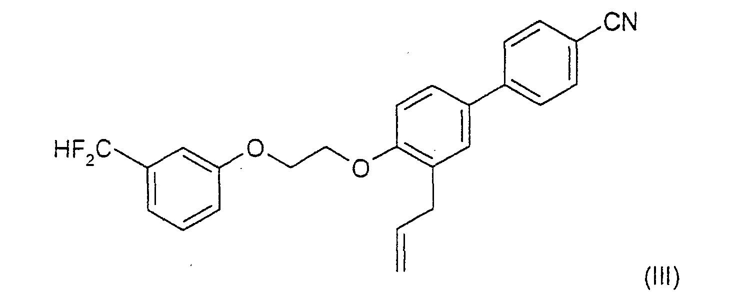

1 g des Nitrils (III) wurden in 20 ml Ethanol vorgelegt und eine Lösung aus 700 mg Hydroxylamin-hydrochlorid, 590 mg Na₂CO₃ in 3 ml H₂O unter Rückfluß langsam zugetropft. Anschließend wurde 4 h gekocht und nach Abkühlen die ausgefallenen Kristalle abgesaugt und mit H₂O nachgewaschen. Nach Neutralisation mit 260 mg Methansulfonsäure in 5 ml Ethanol wurde mit Diethylether gefällt und abgesaugt. Die Substanz wurde in 50 ml Methanol unter Zugabe von 200 mg Pd/C (5%) bei Normaldruck hydriert. Nach Absaugen des Katalysators wurden Raney-Ni zugesetzt und nochmals hydriert. Der Katalysator wurde abgesaugt, das Lösungsmittel abdestilliert, der Rückstand in wenig Ethanol gelöst und mit Diethylether gefällt Ausbeute: 830 mg. Fp.: 204-205°C (als Methansulfonat).

## Patentansprüche

1. Verbindung der Formel (I) 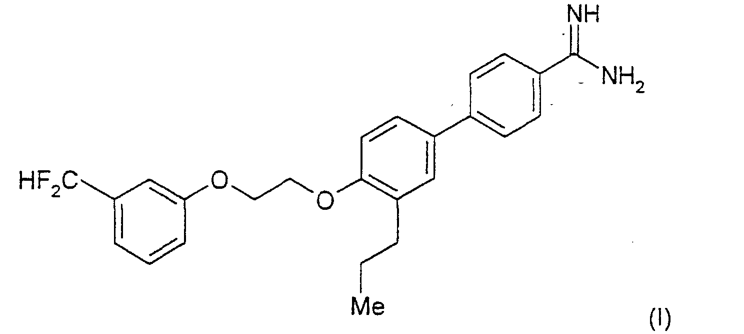 gegebenenfalls in Form ihrer pharmakologisch unbedenklichen Säureadditionssalze.

2. Verfahren zur Herstellung der Verbindung der Formel (I), **dadurch gekennzeichnet, daß** man ein Nitril der Formel (II) 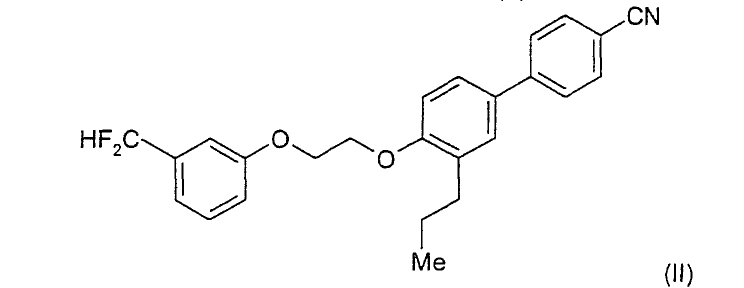 auf an sich bekannte Weise in die Verbindung der Formel (I) überführt.

3. Verfahren zur Herstellung der Verbindung der Formel (I), **dadurch gekennzeichnet, daß** man ein Nitril der Formel (III) 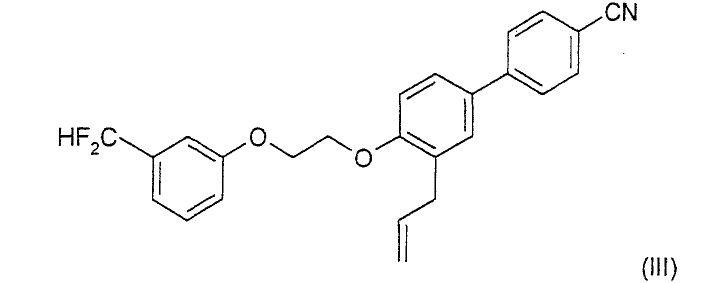 auf an sich bekannte Weise in die Verbindung der Formel (I) überführt.

4. Pharmazeutische Zubereitung, **gekennzeichnet durch** einen Gehalt an einer Verbindung der Formel (I) nach Anspruch 1 neben üblichen Hilfs- und Trägersstoffen.

5. Verwendung einer Verbindung der Formel (I) nach Anspruch 1 zur Herstellung eines Arzneimittels mit LTB₄-antagonistischer Wirkung.

6. Verwendung einer Verbindung der Formel (I) nach Anspruch 1, zur Herstellung eines Medikaments zur therapeutischen Behandlung von Arthritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/Ischämien, Atheriosklerose, multipler Sklerose.

## Claims

1. Compound of formula (I) 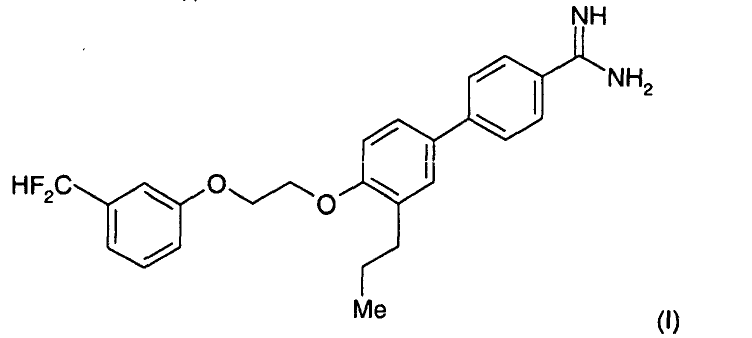 optionally in the form of the pharmacologically acceptable acid addition salts thereof.

2. Process for preparing the compound of formula (I), **characterised in that** a nitrile of formula (II) 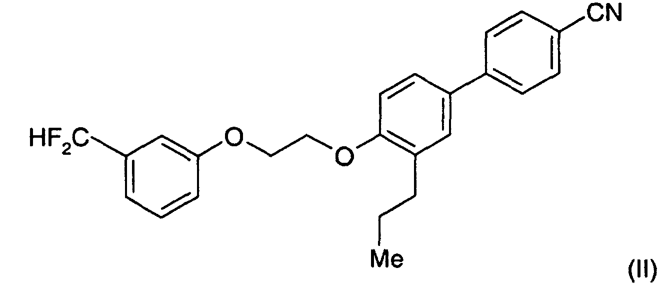 is converted into the compound of formula (I) in a manner known *per se.*

3. Process for preparing the compound of formula (I), **characterised in that** a nitrile of formula (III) 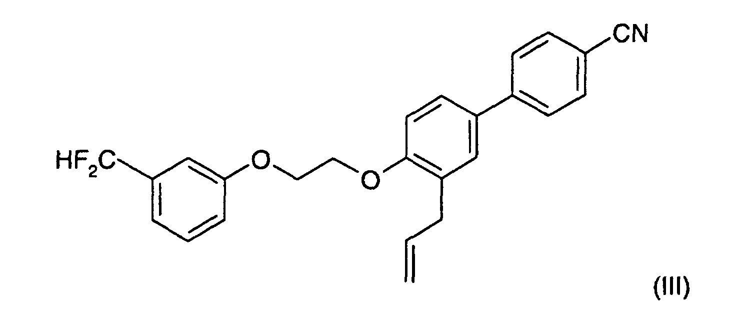 is converted into the compound of formula (I) in a manner known *per se.*

4. Pharmaceutical preparation, **characterised in that** it contains a compound of formula (I) according to claim 1 together with conventional excipients and carriers.

5. Use of a compound of formula (I) according to claim 1 for preparing a pharmaceutical composition with an LTB₄-antagonistic activity.

6. Use of a compound of formula (I) according to claim 1, for preparing a medicament for the therapeutic treatment of arthritis, asthma, chronic obstructive lung diseases such as chronic bronchitis, psoriasis, ulcerative colitis, gastro- or enteropathy induced by nonsteroidal antiphlogistics, cystic fibrosis, Alzheimer's disease, shock, reperfusion damage/ischaemia, atherosclerosis, multiple sclerosis.

## Revendications

1. Composé de formule (I) 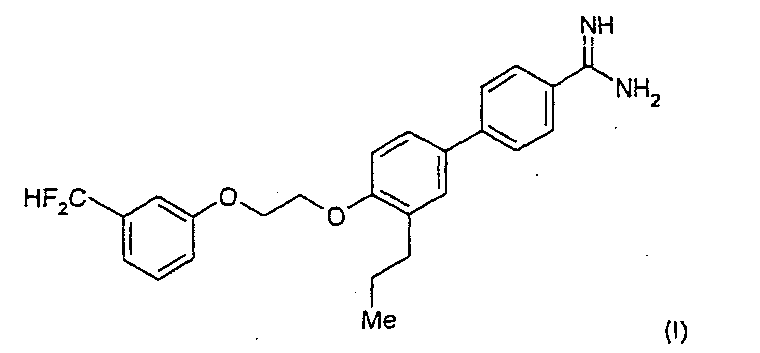 éventuellement sous forme de ses sels d'addition d'acide pharmacologiquement acceptables.

2. Procédé de préparation du composé de formule (I) **caractérisé en ce que** l'on convertit un nitrile de formule (II) 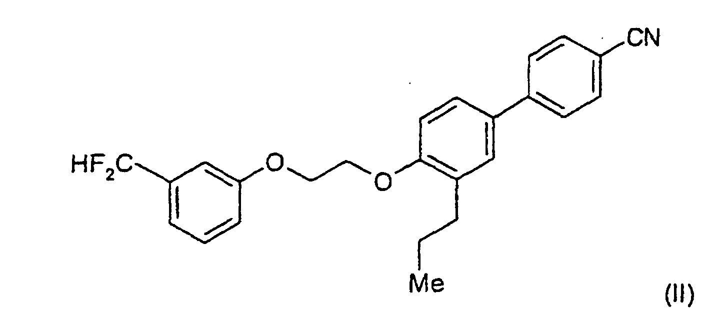 de manière connue en soi en le composé de formule (I).

3. Procédé de préparation du composé de formule (I) **caractérisé en ce que** l'on convertit un nitrile de formule (III) 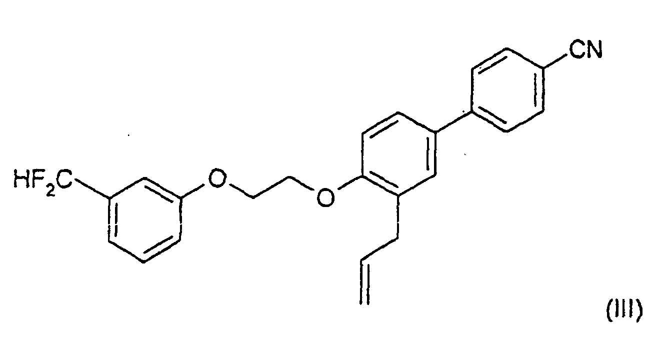 de manière connue en soi en le composé de formule (I).

4. Préparation pharmaceutique **caractérisée par** une teneur en un composé de formule (I) selon la revendication 1, outre des adjuvants et supports courants.

5. Utilisation d'un composé de formule (I) selon la revendication 1 pour la production d'un médicament ayant un effet antagoniste de LTB₄.

6. Utilisation d'un composé de formule (I) selon la revendication 1 pour la production d'un médicament pour le traitement thérapeutique de l'arthrite, de l'asthme, des maladies pulmonaires obstructives chroniques comme la bronchite chronique, du psoriasis, de la colite ulcéreuse, de la gastro- ou entéropathie induite par des antiphlogistiques non stéroïdiens, de la fibrose kystique, de la maladie d'Alzheimer, du choc, des lésions de reperfusion/ischémies, de l'athérosclérose, de la sclérose en plaques.
